(19) Europäisches Patentamt
European Patent Office
Office européen des brevets

(11) **EP 1 379 882 B1**

(12) **EUROPEAN PATENT SPECIFICATION**

(45) Date of publication and mention
of the grant of the patent:
**16.07.2008 Bulletin 2008/29**

(51) Int Cl.:
*G01N 33/68* (2006.01)    *A61P 25/28* (2006.01)
*C07K 14/47* (2006.01)    *A01K 67/027* (2006.01)

(21) Application number: **02740468.0**

(22) Date of filing: **13.04.2002**

(86) International application number:
**PCT/EP2002/004136**

(87) International publication number:
**WO 2002/084297 (24.10.2002 Gazette 2002/43)**

(54) **DIAGNOSTIC AND THERAPEUTIC USE OF A NUCLEAR RESTRICTED PROTEIN FOR ALZHEIMER'S DISEASE AND RELATED NEURODEGENERATIVE DISORDERS**

VERWENDUNG EINES ZUM KERN BESCHRÄNKTEN PROTEIN FÜR DIE DIAGNOSE UND THERAPIE VON ALZHEIMER-KRANKHEIT UND RELATIERTE NEURODEGENERATIEFE STÖRUNGEN

UTILISATION A DES FINS DIAGNOSTICS ET THERAPEUTIQUES D'UNE PROTEINE NUCLEAIRE RESTREINTE POUR LA MALADIE D'ALZHEIMER ET DES TROUBLES NEURODEGENERATIFS ASSOCIES

(84) Designated Contracting States:
**AT BE CH CY DE DK ES FI FR GB GR IE IT LI LU MC NL PT SE TR**

(30) Priority: **14.04.2001 EP 01109230**

(43) Date of publication of application:
**14.01.2004 Bulletin 2004/03**

(73) Proprietor: **EVOTEC Neurosciences GmbH**
**22525 Hamburg (DE)**

(72) Inventors:
• **HIPFEL, Rainer,**
  **69120 Heidelberg (DE)**
• **Von der KAMMER, H.,**
  **22607 Hamburg (DE)**
• **POHLNER, Johannes,**
  **22175 Hamburg (DE)**

(74) Representative: **Meyers, Hans-Wilhelm et al**
**Patentanwälte**
**von Kreisler-Selting-Werner**
**Postfach 10 22 41**
**50462 Köln (DE)**

(56) References cited:
**WO-A-98/28418        WO-A-99/01764**
**US-A- 5 932 475**

• **WOLFE ANDREW M ET AL: "Cell-specific expression of the human gonadotropin-releasing hormone gene in transgenic animals." JOURNAL OF BIOLOGICAL CHEMISTRY, vol. 271, no. 33, 1996, pages 20018-20023, XP002187065 ISSN: 0021-9258**
• **IM SUHN-YOUNG ET AL: "Mechanisms for multiple activation of NF-kappaB." FASEB JOURNAL, vol. 15, no. 4, 7 March 2001 (2001-03-07), page A697 XP002187066 Annual Meeting of the Federation of American Societies for Experimental Biology on Experimental Biology 2001;Orlando, Florida, USA; March 31-April 04, 2001 ISSN: 0892-6638**
• **KIM TAE-AUG ET AL: "Genomic organization, chromosomal localization and regulation of expression of the neuronal nuclear matrix protein NRP/B in human brain tumors." GENE (AMSTERDAM), vol. 255, no. 1, 2000, pages 105-116, XP004208816 ISSN: 0378-1119**
• **AGUZZI ADRIANO ET AL: "Transgenic and Knock-out Mice: Models of Neurological Disease." BRAIN PATHOLOGY, vol. 4, no. 1, 1994, pages 3-20, XP000561411 ISSN: 1015-6305**

EP 1 379 882 B1

- **KIM TAE-AUG ET AL: "NRP/B, a novel nuclear matrix protein, associates with p110RB and is involved in neuronal differentiation" JOURNAL OF CELL BIOLOGY, ROCKEFELLER UNIVERSITY PRESS, NEW YORK, US, US, vol. 141, no. 3, 4 May 1998 (1998-05-04), pages 553-566, XP002146073 ISSN: 0021-9525 cited in the application**

**Description**

The present invention relates to methods of diagnosing, prognosticating and monitoring the progression of Alzheimer's disease in a subject. Furthermore, methods of therapy control and screening for modulating agents of neurodegenerative diseases are provided.

Neurodegenerative diseases, in particular Alzheimer's disease (AD), have a strongly debilitating impact on a patient's life. Furthermore, these diseases constitute an enormous health, social and economic burden. AD is the most common age-related neurodegenerative condition affecting about 10 % of the population over 65 years of age and up to 45 % over age 85 (for a recent review see Vickers et al., Progress in Neurobiology 2000, 60:139-165). Presently, this amounts to an estimated 12 million cases in the US, Europe, and Japan. This situation will inevitably worsen with the demographic increase in the number of old people ("aging of the baby boomers") in developed countries. The neuropathological hallmarks that occur in the brains of individuals with AD are senile plaques, composed of amyloid-b protein, and profound cytoskeletal changes coinciding with the appearance of abnormal filamentous structures and the formation of neurofibrillary tangles. AD is a progressive disease that is associated with early deficits in memory formation and ultimately leads to the complete erosion of higher cognitive function. A characteristic feature of the pathogenesis of AD is the selective vulnerability of particular brain regions and subpopulations of nerve cells to the degenerative process. Specifically, the temporal lobe region and the hippocampus are affected early and more severely during the progression of the disease. On the other hand, neurons within the frontal cortex, occipital cortex, and the cerebellum remain largely intact and are protected from neurodegeneration (Terry et al., Annals of Neurology 1981, 10:184-192).

Currently, there is no cure for AD, nor is there an effective treatment to halt the progression of AD or even a method to diagnose AD antemortem with high probability. Several risk factors have been identified that predispose an individual to develop AD, among them most prominently the epsilon4 allele of apolipoprotein E (ApoE). Although there are rare examples of early-onset AD which have been attributed to genetic defects in the genes for the amyloid precursor protein (APP), presenilin-1, and presenilin-2, the prevalent form of late-onset sporadic AD is of hitherto unknown etiologic origin. The late onset and complex pathogenesis of neurodegenerative disorders pose a formidable challenge to the development of therapeutic and diagnostic agents. It is crucial to expand the pool of potential drug targets and diagnostic markers. It is therefore an object of the present invention to provide methods, materials, and animal models which are suited inter alia for the diagnosis and development of a treatment of AD or related neurodegenerative diseases. This object has been solved by the features of the independent claims. The subclaims define preferred embodiments of the present invention.

The gene coding for the nuclear restricted protein/brain (NRP/B) was originally cloned by Kim et al. (Journal of Cell Biology 1998, 141:553-566) in an attempt to identify proteins that may play a role in brain development and neuronal differentiation. The human NRP/B gene (Genbank Accession No. NM 003633) codes for a 589-amino acid protein with a predicted molecular weight of 67 kDa. NRP/B shares no homology or similarity to any other known proteins, and its amino acid sequence is highly conserved between human and mouse. At its N-terminus the NRP/B protein possesses a BTB domain-like structure (~35% identity) which has been implicated in protein-protein interactions involving the cytoskeleton. The C-terminus shows some homology (~28% identity) to a "kelch motif" which is shared among several actin-associated proteins. The NRP/B gene is expressed as a 5.5 kb mRNA predominantly in human fetal and adult brain with modest expression in a few other fetal tissues such as heart, kidney, and lung, and very low levels of expression in adult pancreas. In the human adult brain, expression is particularly prominent in the hippocampus, amygdala, and cerebral cortex. The NRP/B protein can exist in two forms. A 67 kDa form and a 57 kDa form are detected in total cell lysates, whereas in the nuclear fraction only the 67 kDa form is observed. In the nucleus NRP/B seems to be associated with the nuclear matrix.

A functional analysis of NRP/B by Kim et al. (ref, see above) suggests a participation of NRP/B in the regulation of neuronal differentiation and process formation. This notion is based on the finding that NRP/B expression is up-regulated during neuronal differentiation, and that overexpression of NRP/B augments neuronal process formation in cell culture experiments. Furthermore, NRP/B antisense inhibition experiments in rat primary hippocampal neurons impedes neurite development. Another functional aspect of NRP/B is its physical association with the functionally active, hypophosphorylated form of the p110$^{RB}$ retinoblastoma protein during neuronal differentiation of human SH-SY5Y neuroblastoma cells induced by retinoic acid. The hypophosphorylated form of p110$^{RB}$ is also found to be associated with the nuclear matrix, and overexpression of p110$^{RB}$ can induce neuronal differentiation. Thus, NRP/B could play a potential role in the regulation of the cell cycle by interfering with other cell cycle regulatory proteins such as p110$^{RB}$. This invention is based on the differential expression of the gene coding for NRP/B in brain samples of AD patients. More specifically, the present invention discloses a differential up-regulation of NRP/B gene expression in the frontal lobe region of AD patients relative to samples derived from the temporal cortex region. No such up-regulation is observed in samples from age-matched healthy controls. To date, no experiments have been described that show a relationship between a differential expression of the gene coding for NRP/B and the pathology of neurodegenerative diseases, particularly AD. Such a link offers new ways, inter alia, for the diagnosis and treatment of said disorders.

The singular forms "a", "an", and "the" as used herein and in the claims include plural reference unless the context dictates otherwise. For example, "a cell" means as well a plurality of cells, and so forth. The term "and/or" as used in the present specification and in the claims implies that the phrases before and after this term are to be considered either as alternatives or in combination. For instance, the wording "determination of a level and/or an activity" means that either only a level, or only an activity, or both a level and an activity are determined. The term "level" as used herein is meant to comprise a gage of, or a measure of the amount of, or a concentration of a transcription product, for instance an mRNA, or a translation product, for instance a protein or polypeptide. The term "activity" as used herein shall be understood as a measure for the ability of a transcription product or a translation product to produce a biological effect or a measure for a level of biologically active molecules. The term "activity" also refers to enzymatic activity. The terms "level" and/or "activity" as used herein further refer to gene expression levels or gene activity. Gene expression can be defined as the utilization of the information contained in a gene by transcription and translation leading to the production of a gene product. A gene product comprises either RNA or protein and is the result of expression of a gene. The amount of a gene product can be used to measure how active a gene is. The term "gene" as used in the present specification and in the claims comprises both coding regions (exons) as well as non-coding regions (e.g. non-coding regulatory elements such as promoters or enhancers, introns, leader and trailer sequences). The term "fragment" as used herein is meant to comprise e.g. an alternatively spliced, or truncated, or otherwise cleaved transcription product or translation product. The term "derivative" as used herein refers to a mutant, or an RNA-edited, or a chemically modified, or otherwise altered transcription product, or to a mutant, or chemically modified, or otherwise altered translation product. For instance, a "derivative" may be generated by processes such as altered phosphorylation, or glycosylation, or lipidation, or by altered signal peptide cleavage or other types of maturation cleavage. These processes may occur post-translationally. The term "modulator" as used in the present invention and in the claims refers to a molecule capable of changing or altering the level and/or the activity of a gene, or a transcription product of a gene, or a translation product of a gene. Preferably, a "modulator" is capable of changing or altering the biological activity of a transcription product or a translation product of a gene. Said modulation, for instance, may be an increase or a decrease in enzyme activity, a change in binding characteristics, or any other change or alteration in the biological, functional, or immunological properties of said translation product of a gene. The term 'AD' shall mean Alzheimer's disease.

Neurodegenerative diseases or disorders according to the present invention comprise Alzheimer's disease, Parkinson's disease, Huntington's disease, amyotrophic lateral sclerosis, Pick's disease, fronto-temporal dementia, progressive nuclear palsy, corticobasal degeneration, cerebrovascular dementia, multiple system atrophy, and mild-cognitive impairment. Further conditions involving neurodegenerative processes are, for instance, ischemic stroke, age-related macular degeneration and narcolepsy.

In one aspect, the invention features a method of diagnosing or prognosticating Alzheimer's disease in a subject, or determining whether a subject is at Increased risk of developing Alzheimer's disease, comprising determining up-regulation of

    (i) a transcription product of a gene coding for NRP/B, and/or
    (ii) a translation product of a gene coding for NRP/B, and/or
    (iii) a fragment or mutant, RNA edited or chemically modified derivative of said transcription or translation product,

In a brain tissue or cerebrospinal fluid sample from said subject or up-regulation of NRP/B in the frontal cortex relative to the temporal cortex from said subject and comparing said level to a reference value representing a known disease or health status, thereby diagnosing or prognosticating Alzheimer's disease in said subject, or determining whether said subject is at increased risk of developing Alzheimer's disease.

In a further aspect, the invention features a method of monitoring the progression of Alzheimer's disease in a subject, comprising determining up-regulation of

    (i) a transcription product of a gene coding for NRP/B, and/or
    (ii) a translation product of a gene coding for NRP/B, and/or
    (iii) a fragment or mutant, RNA edited or chemically modified derivative of said transcription or translation product,

in a brain tissue or cerebrospinal fluid sample from said subject or up-regulation of NRP/B in the frontal cortex relative to the temporal cortex from said subject and comparing said level to a reference value representing a known disease or health status, thereby monitoring the progression of Alzheimer's disease in said subject.

In still a further aspect, the invention features a method of evaluating a treatment for Alzheimer's disease, comprising determining up-regulation of

    (i) a transcription product of a gene coding for NRP/B, and/or
    (ii) a translation product of a gene coding for NRP/B, and/or

(iii) a fragment or mutant, RNA edited or chemically modified derivative of said transcription or translation product,

in a brain tissue or cerebrospinal fluid sample from a subject or up-regulation of NRP/B in the frontal cortex relative to the temporal cortex from said subject being treated for Alzheimer's disease and comparing said level to a reference value representing a known disease or health status, thereby evaluating said treatment for Alzheimer's disease.

The present invention discloses also the differential expression and regulation of the gene coding for the nuclear restricted protein / brain in specific brain regions of AD patients. Consequently, the NRP/B gene and its corresponding translation products may have a causative role in the regional selective neuronal degeneration typically observed in AD. Alternatively, NRP/B may confer a neuroprotective function to the remaining surviving nerve cells. Based on these disclosures, the present invention discloses utility for the diagnostic evaluation and prognosis as well as for the identification of a predisposition to AD. Furthermore, the present invention is useful for the diagnostic monitoring of patients undergoing treatment for such a disease.

The sample of a subject to be analyzed and determined is selected from the group consisting of a brain tissue or cerebrospinal fluid.

The reference value is that of a level, of (i) a transcription product of a gene coding for a nuclear restricted protein, and/or of (ii) a translation product of a gene coding for a nuclear restricted protein, and/or of (iii) a fragment or mutant, RNA edited or chemically modified derivative of said transcription or transla-tion product in a sample from a subject not suffering from Alzheimer's disease.

An increase of a transcription product of a gene coding for NRP/B and/or a translation product of a gene coding for NRP/B in a sample cell or tissue from said subject relative to a reference value representing a known health status indicates a diagnosis, or prognosis, or increased risk of becoming diseased with Alzheimer's disease.

In preferred embodiments, measurement of a level of transcription products of a gene coding for a nuclear restricted protein is performed in a sample from a subject using a quantitative PCR-analysis with primer combinations to amplify said gene specific sequences from cDNA obtained by reverse transcription of RNA extracted from a sample of a subject. A Northern blot with probes specific for said gene can also be applied. It might further be preferred to measure transcription products by means of chip-based micro-array technologies. These techniques are known to those of ordinary skill in the art (see Sambrook and Russell, Molecular Cloning: A Laboratory Manual, Cold Spring Harbor Laboratory Press, Cold Spring Harbor, New York, 2000).

Furthermore, a level of a translation product of a gene coding for NRP/B and/or fragment of said translation product can be detected using an immunoassay, and/or binding assay. These assays can measure the amount of binding between said protein molecule and an anti-protein antibody by the use of enzymatic, chromodynamic, radioactive, magnetic, or luminescent labels which are attached to either the anti-protein antibody or a secondary antibody which binds the anti-protein antibody. In addition, other high affinity ligands may be used. Immunoassays which can be used include e.g. ELISAs, Western blots and other techniques known to those of ordinary skill In the art (see Harlow and Lane, Antibodies: A Laboratory Manual, Cold Spring Harbor Laboratory Press, Cold Spring Harbor, New York, 1999). All these detection techniques may also be employed in the format of micro-arrays, protein-arrays, or protein-chip based technologies.

In a preferred embodiment, the level of (i) a transcription product of a gene coding for NRP/B, and/or of (ii) a translation product of a gene coding for NRP/B; and/or of (iii) a fragment or mutant, RNA edited or chemically modified derivative of said transcription or translation product in a series of samples taken from said subject over a period of time is compared, in order to monitor the progression of said disease. In further preferred embodiments, said subject receives a treatment prior to one or more of said sample gatherings. In yet another preferred embodiment, said level is determined before and after said treatment of said subject.

In another aspect, the invention features an assay for screening for a modulator of AD of one or more substances selected from the group consisting of (i) a gene coding for NRP/B and/or (ii) a transcription product of a gene coding for NRP/B, and/or (iii) a translation product of a gene coding for NRP/B, and/or (iv) a fragment or mutant, RNA edited or chemically modified derivative of (i) to (iii). This screening method comprises (a) contacting a cell with a test compound, and (b) measuring the levels of one or more substances recited in (i) to (iv), and (c) measuring the levels, of said substances in a control cell not contacted with said test compound, and (d) comparing the levels of the substance in the cells of step (b) and (c), wherein an alteration in the level of said substances in the contacted cells indicates that the test compound is a modulator of AD.

In one further aspect, the invention features a screening assay for a modulator of AD of one or more substances selected from the group consisting of (i) a gene coding for NRP/B and/or (ii) a transcription product of a gene coding for NRP/B, and/or (iii) a translation product of a gene coding for NRP/B, and/or (iv) a fragment or mutant, RNA edited or chemically modified derivative of (i) to (iii), comprising (a) administering a test compound to a test animal which is predisposed to developing or has already developed AD, and (b) measuring the level of one or more substances recited in (i) to (iv), and (c) measuring the level of said substances in a matched control animal which is equally predisposed to developing or has already developed AD and to which animal no such test compound has been administered, and (d) comparing the level of the substance in the animals of step (b) and (c), wherein an alteration in the level of substances in the test animal indicates that the test compound is a modulator of AD, wherein the test animal excludes man.

In a preferred embodiment, said test animal and/or said control animal is a recombinant, non-human animal which expresses NRP/B stricted protein, or a fragment thereof, or a mutant, RNA edited or chemically modified derivative thereof, under the control of a transcriptional regulatory element which is not the native NRP/B gene transcriptional control regulatory element.

The present invention features the use of an antibody which is specifically immunoreactive with an immunogen, wherein said immunogen is a translation product of a gene coding for NRP/B, or a fragment thereof. The immunogen may comprise immunogenic or antigenic epitopes or portions of a translation product of said gene, wherein said immunogenic or antigenic portion of a translation product is a polypeptide, and wherein said polypeptide elicits an antibody response in an animal, and wherein said polypeptide is immunospecifically bound by said antibody. Methods for generating antibodies are well known in the art (see Harlow et al., Antibodies, A Laboratory Manual, Cold Spring Harbor Laboratory Press, Cold Spring Harbor, New York). The term "antibody", as employed in the present invention, encompasses all forms of antibodies known in the art, such as polyclonal, monoclonal, chimeric, recombinatorial, anti-idiotypic, humanized, or single chain antibodies, as well as fragments thereof. Antibodies of the present invention are useful, for instance, in a variety of diagnostic and therapeutic methods involving detecting translation products of a gene coding for the nuclear restricted protein / brain, NRP/B.

In a preferred embodiment of the present invention, said antibodies can be used for detecting the pathological state of a cell in a sample from a subject, comprising immunocytochemical staining of said cell with said antibody, wherein an altered degree of staining, in said cell compared to a cell representing a known health status indicates a pathological state of said cell, wherein the pathological state relates to AD. Immunocytochemical staining of a cell can be carried out by a number of different experimental methods well known in the art. It might be preferred, however, to apply an automated method for the detection of antibody binding, wherein the determination of the degree of staining of a cell, or the determination of the cellular or subcellular staining pattern of a cell, or the topological distribution of an antigen on the cell surface or among organelles and other subcellular structures within the cell, are carried out according to the method described in US patent 6150173).

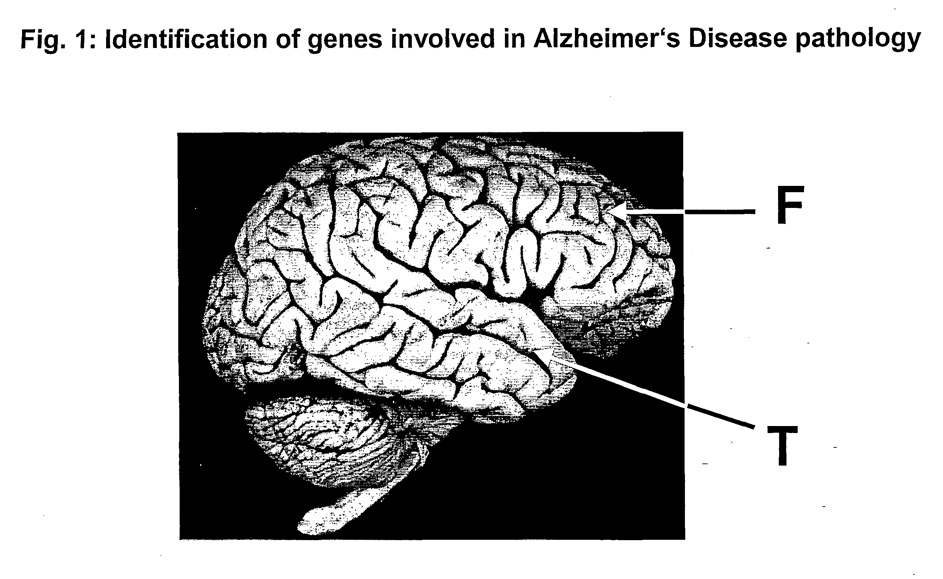

Figure 1 depicts the brain regions with selective vulnerability to neuronal loss and degeneration in AD. Primarily, neurons within the inferior temporal lobe, the entorhinal cortex, the hippocampus, and the amygdala are subject to degenerative processes in AD (Terry et al., Annals of Neurology 1981, 10:184-192). These brain regions are mostly involved in the processing of learning and memory functions. In contrast, neurons within the frontal cortex, the occipital cortex, and the cerebellum remain largely intact and preserved from neurodegenerative processes in AD. Brain tissues from the frontal cortex (F) and the temporal cortex (T) of AD patients and healthy, age-matched control individuals was used for the herein disclosed examples. For illustrative purposes, the image of a healthy brain was taken from a publication by Strange (Brain Biochemistry and Brain Disorders, Oxford University Press, Oxford, 1992, p.4).

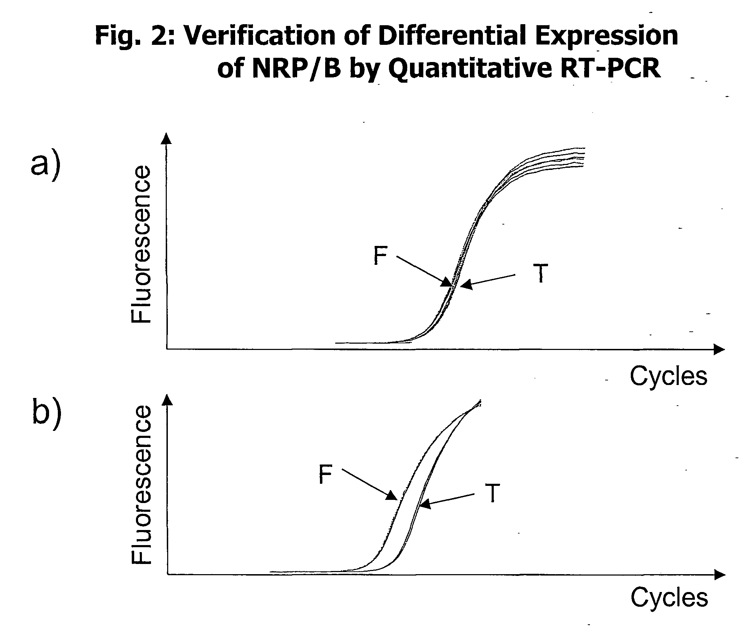

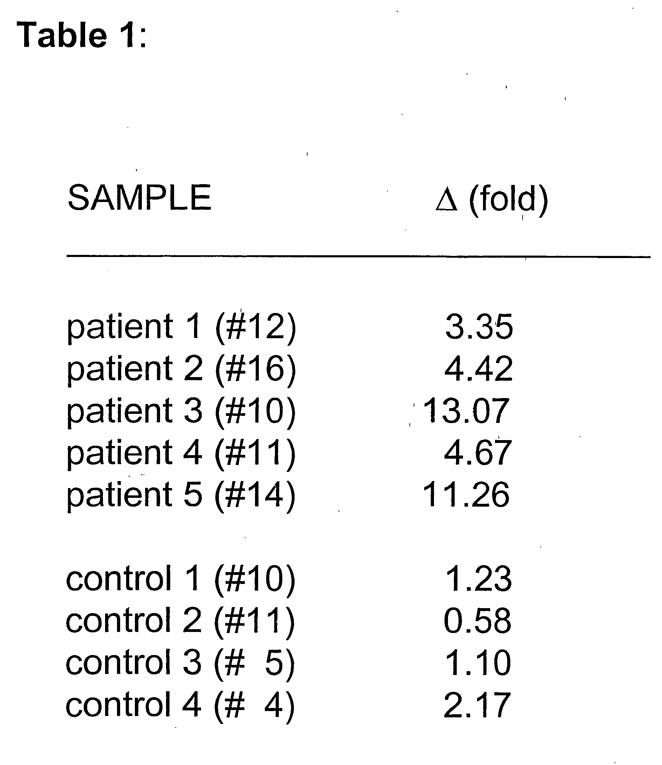

Figure 2 illustrates the verification of the differential expression of NRP/B by quantitative RT-PCR analysis. Quantification of RT-PCR products from RNA samples collected from the frontal cortex (F) and temporal cortex (T) of healthy, age-matched control individuals (Fig 2a) and AD patients (Fig 2b) was performed by the LightCycler rapid thermal cycling technique. The data were normalized to cyclophilin B which showed no significant difference in its gene expression level. The figure depicts the kinetics of amplification by plotting the cycle number against the amount of amplified material as measured by its fluorescence. The amplification kinetics of NRP/B cDNA from both the frontal and temporal cortices of a normal control individual during the exponential phase of the reaction overlap (Fig 2a), whereas in AD there is a significant shift of the curve for the sample derived from frontal cortex (Fig 2b), indicating an up-regulation of NRP/B mRNA in the frontal cortex relative to temporal cortex.

Table 1 lists the gene expression levels in the frontal cortex relative to the temporal cortex for the gene coding for the nuclear restricted protein / brain, NRP/B, in five AD patients (3.35 to 13.07 fold) and four healthy, age-matched control individuals (0.58 to 2.17 fold). The values shown are reciprocal values according to the formula described herein (see below).

EXAMPLE I:

(i) Brain tissue dissection from patients with AD:

Brain tissues from AD patients and age-matched control subjects were obtained from qualified institutions and brain banks. The tissue was collected within 6 hours post-mortem and immediately frozen on dry ice. Sample sections from each tissue were fixed in paraformaldehyde for histo-pathological confirmation of the diagnosis. Brain areas for differential expression analysis were identified (see Fig. 1) and stored at -80 °C until RNA extractions were performed.

(ii) Isolation of total mRNA:

Total RNA was extracted from post-mortem brain tissue by using the RNeasy kit (Qiagen) according to the manufacturer's protocol. The quality of the prepared RNA was determined by formaldehyde agarose gel electrophoresis and Northern blotting according to standard procedures (Sambrook and Russell, Molecular Cloning: A Laboratory Manual, Cold Spring Harbor Laboratory Press, Cold Spring Harbor, New York, 2000). The mRNA was isolated from the total RNA preparation using the Quick-prep Micro mRNA Purification Kit (Pharmacia Biotech).

(iii) cDNA synthesis and identification of differentially expressed genes by suppressive subtractive hybridization:

This technique compares two populations of mRNA and provides clones of genes that are expressed in one population but not in the other. The applied technique was described in detail by Diatchenko et al. (Proc Natl Acad Sci USA 1996, 93:6025-30). In the present invention, mRNA populations from post-mortem brain tissues from AD patients were compared. Specifically, mRNA of the frontal cortex was subtracted from mRNA of the inferior temporal cortex. The necessary reagents were taken from the PCR-Select cDNA subtraction kit (Clontech), and all steps were performed as described in the manufacturer's protocol. Specifically, $2\mu$g mRNA each were used for first-strand and second-strand cDNA synthesis. After RsaI-digestion and adaptor ligation hybridization of tester and driver was performed for 8 hours (first hybridization) and 15 hours (second hybridization) at 68 °C. Two PCR steps were performed to amplify differentially expressed genes (first PCR: 27 cycles of 94 °C and 30 sec, 66 °C and 30 sec, and 72 °C and 1.5 min; nested PCR: 12 cycles of 94°C and 30 sec, 66 °C and 30 sec, and 72 °C and 1.5 min) using adaptor specific primers (included in the subtraction kit) and 50x Advantage Polymerase Mix (Clontech). Efficiencies of RsaI-digestions, adaptor ligations and subtractive hybridizations were checked as recommended in the kit. Subtracted cDNAs were inserted into the pCRe vector and transformed into *E.coli* INVaF' cells (Invitrogen). To isolate individual cDNAs of the subtracted library single bacterial transformants were incubated in 100 $\mu$l LB (with 50 $\mu$g/ml ampicillin) at 37 °C for at least 4 hours. Inserts were PCR amplified (95 °C and 30 sec, 68 °C and 3 min for 30 cycles) in a volume of 20 $\mu$l containing 10 mM Tris-HCl pH 9.0, 1.5 mM $MgCl_2$, 50 mM KCl, 200 $\mu$M dNTP, 0.5 $\mu$M adaptor specific primers (included in the subtraction kit), 1.5 Units Taq polymerase (Pharmacia Biotech), and 1$\mu$l of bacterial culture. An aliquot of the mixture (1.5 $\mu$l) containing 3 $\mu$l PCR amplified inserts and 2 $\mu$l 0.3 N NaOH/15% Ficoll were spotted onto a positively charged nylon membrane (Roche). In this way, hundreds of spots were arrayed on duplicate filters for subsequent hybridization. The differential screening step consisted of hybridizations of the subtracted library with itself to minimize background (Wang and Brown, Proc Natl Acad Sci USA 1991, 88:11505-9). The probes were made of the nested PCR product of the subtraction following the instructions of the Clontech subtraction kit. Labeling with digoxigenin was performed with the DIG DNA Labeling Kit (Roche). Hybridizations were carried out overnight in DIG Easy HYB (Roche) at 43°C. The filters were washed twice in 2 x SSC / 0.5 % SDS at 68 °C for 15 min and twice in 0.1 x SSC / 0.5 % SDS at 68 °C for 15 min, and subjected to detection using anti-DIG-AP conjugates and CDP-Star™ as chemiluminescent substrate according to the instructions of the DIG DNA Detection Kit (Roche). Blots were exposed to Kodak Bio-max MR chemiluminescent film at room temperature for several minutes. The nucleotide sequences of clones of interest were obtained using methods well known in the art. For nucleotide sequence analyses and homology searches, computer algorithms of the University of Wisconsin Genetics Computer Group (GCG) together with publicly available nucleotide and peptide sequence information (GenBank and EMBL databases) were employed.

(iv) Confirmation of differential expression by quantitative RT-PCR:

Positive confirmation of differential expression of the NRP/B gene was performed using the LightCycler technology (Roche). This technique features rapid thermal cyling for the polymerase chain reaction as well as real-time measurement of fluorescent signals during amplification and therefore allows for highly accurate quantification of RT-PCR products by using a kinetic, rather than an endpoint approach. The ratio of NRP/B cDNA from the temporal cortex and frontal cortex was determined (relative quantification). In a first step a standard curve was generated to determine the efficiency of the PCR with specific primers for NRP/B (5'-ATAGGTGCTTCCCCTGAGGTG-3' and 5'-GCAATGTGAGAAACATGGACGA-3'). PCR amplification (95 °C and 1 sec, 56 °C and 5 sec, and 72 °C and 5 sec) was performed in a volume of 20 $\mu$l containing Lightcycler-DNA Master SYBR Green mix (containing Taq DNA polymerase, reaction buffer, dNTP mix with dUTP instead of dTTP, SYBR Green I dye, and 1 mM $MgCl_2$, Roche), additionally containing 3 mM $MgCl_2$, 0,5 $\mu$M primers, 0,16 $\mu$l TaqStart® antibody (Clontech), and 1 $\mu$l of a cDNA dilution series (40, 20, 10, 5, and 1 ng human total brain cDNA, Clontech). Melting curve analysis revealed a single peak at approximately 83°C with no visible primer dimers. Agarose gel analysis of the PCR product showed one single band of the expected size (78 bp).

The same protocol was applied to determine the PCR efficiency of the reference gene, cyclophilin B, using the specific primers 5'-ACTGAAGCACTACGGGCCTG-3' and 5'-AGCCGTTGGTGTCTTTGCC-3' except for $MgCl_2$ (an additional 1 mM was added instead of 3 mM). Cyclophilin-B was chosen for normalization because it was found to be the least regulated gene among all analyzed housekeeping genes. Melting curve analysis revealed a single peak at approximately 87 °C with no visible primer dimers. Agarose gel analysis of the PCR product showed one single band of the expected size (62 bp). The logarithm of the cDNA concentration was plotted against the threshold cycle number $C_t$ for both NRP/B and cyclophilin B. The slopes and the intercepts of the standard curves (i.e. linear regressions) were calculated for both genes. In a second step, cDNA from temporal cortex and frontal cortex was analyzed in parallel with cyclophilin B for normalization. The $C_t$ values were measured and converted to ng total brain cDNA using the corresponding standard curves:

$$10 \wedge ( (C_t \text{ value} - \text{intercept}) / \text{slope} ) \quad [\text{ng total brain cDNA}]$$

The values of temporal and frontal cortex NRP/B cDNAs were normalized to cyclophilin B and the ratio was calculated using the following formula:

$$\text{Ratio} = \frac{\text{NRP/B temporal [ng] / cyclophilin B temporal [ng]}}{\text{NRP/B frontal [ng] / cyclophilin B frontal [ng]}}$$

The results of one such quantitative RT-PGR analysis for the NRP/B gene are shown in Fig. 2.

## Claims

1. A method of diagnosing or prognosticating Alzheimer's disease in a subject, or determining whether a subject is at increased risk of developing Alzheimer's disease, comprising determining up-regulation of

   (i) a transcription product of a gene coding for NRP/B, and/or
   (ii) a translation product of a gene coding for NRP/B, and/or
   (iii) a fragment or mutant, RNA edited or chemically modified derivative of said transcription or translation product,

   in a brain tissue or cerebrospinal fluid sample from said subject or up-regulation of NRP/B in the frontal cortex relative to the temporal cortex from said subject and comparing said level to a reference value representing a known disease or health status, thereby diagnosing or prognosticating Alzheimer's disease in said subject, or determining whether said subject is at increased risk of developing Alzheimer's disease.

2. A method of monitoring the progression of Alzheimer's disease in a subject, comprising determining up-regulation of

   (i) a transcription product of a gene coding for NRP/B, and/or
   (ii) a translation product of a gene coding for NRP/B, and/or
   (iii) a fragment or mutant, RNA edited or chemically modified derivative of said transcription or translation product,

   in a brain tissue or cerebrospinal fluid sample from said subject or up-regulation of NRP/B in the frontal cortex relative to the temporal cortex from said subject and comparing said level to a reference value representing a known disease or health status, thereby monitoring the progression of Alzheimer's disease in said subject.

3. A method of evaluating a treatment for Alzheimer's disease, comprising determining up-regulation of

   (i) a transcription product of a gene coding for NRP/B, and/or

(ii) a translation product of a gene coding for NRP/B, and/or
(iii) a fragment or mutant, RNA edited or chemically modified derivative of said transcription or translation product,

in a brain tissue or cerebrospinal fluid sample from a subject or up-regulation of NRP/B in the frontal cortex relative to the temporal cortex from said subject being treated for Alzheimer's disease and comparing said level to a reference value representing a known disease or health status, thereby evaluating said treatment for Alzheimer's disease.

4. The method according to any of claims 1 to 3 wherein said reference value is that of a level of

(i) a transcription product of a gene coding for NRP/B, and/or
(ii) a translation product of a gene coding for NRP/B, and/or
(iii) a fragment or mutant, RNA edited or chemically modified derivative of said transcription or translation product,

in a brain tissue or cerebrospinal fluid sample from a subject not suffering from Alzheimer's disease.

5. The method according to any of claims 1 to 4 wherein an increase in a transcription product of the gene coding for NRP/B and/or a translation product of a gene coding for NRP/B in a brain tissue or cerebrospinal fluid, from said subject relative to a reference value representing a known health status indicates a diagnosis, or prognosis, or increased risk of Alzheimer's disease in said subject.

6. The method according to any of claims 1 to 5, further comprising comparing a level of

(i) a transcription product of a gene coding for NRP/B, and/or
(ii) a translation product of a gene coding for NRP/B, and/or
(iii) a fragment or mutant, RNA edited or chemically modified derivative of said transcription or translation product

in a series of brain tissue or cerebrospinal fluid samples taken from said subject over a period of time.

7. An assay for screening for a modulator of Alzheimer's disease of one or more substances selected from the group consisting of

(i) a gene coding for NRP/B, and/or
(ii) a transcription product of a gene coding for NRP/B, and/or
(iii) a translation product of a gene coding for NRP/B, and/or
(iv) a fragment or mutant, RNA edited or chemically modified derivative of (i) to (iii), said method comprising:

(a) contacting a cell with a test compound;
(b) measuring the levels of one or more substances recited in (i) to (iv);
(c) measuring the levels of one or more substances recited in (i) to (iv) in a control cell not contacted with said test compound; and
(d) comparing the levels of the substance in the cells of step (b) and (c), wherein an alteration in the levels of substances in the contacted cells indicates that the test compound is a modulator of Alzheimer's disease.

8. A method of screening for a modulator of Alzheimer's disease of one or more substances selected from the group consisting of

(i) a gene coding for NRP/B, and/or
(ii) a transcription product of a gene coding for NRP/B, and/or
(iii) a translation product of a gene coding for NRP/B, and/or
(iv) a fragment or mutant, RNA edited or chemically modified derivative of (i) to (iii), said method comprising:

(a) administering a test compound to a test animal excluding man which is predisposed to developing or has already developed Alzheimer's disease in respect of the substances recited in (i) to (iv);
(b) measuring the levels of one or more substances recited in (i) to (iv);
(c) measuring the levels of one or more substances recited in (i) or (iv) in a matched control animal excluding man which is predisposed to developing or has already developed Alzheimer's disease in respect to the substances recited in (i) to (iv) and to which animal no such test compound has been administered;
(d) comparing the levels of the substance in the animals of step (b) and (c), wherein an alteration in the level of substances in the test animal excluding man indicates that the test compound is a modulator of Alzheimer's disease.

9. The method according to claim 8 wherein said test animal excluding man and/or said control animal excluding man is a recombinant animal which expresses NRP/B, or a fragment thereof, or a mutant, RNA edited or chemically modified derivative thereof, under the control of a transcriptional control element which is not the native NRP/B gene transcriptional control element.

10. Use of an antibody specifically immunoreactive with an immunogen, wherein said immunogen is a translation product of a gene coding for NRP/B, or a fragment thereof, for detecting the pathological state of a cell in a brain tissue or cerebrospinal fluid sample from a subject, comprising immunocytochemical staining of said cell with said antibody, wherein an altered degree of staining compared to a cell representing a known health status indicates a pathological state of said cell, and wherein said pathological state relates to Alzheimer's disease.

**Patentansprüche**

1. Verfahren zum Diagnostizieren oder Prognostizieren der Alzheimer-Krankheit bei einem Patienten oder zum Bestimmen, ob ein Patient ein erhöhtes Risiko hat, Alzheimer-Krankheit zu entwickeln, umfassend das Bestimmen der Hochregulierung von:

    (i) einem Transcriptionsprodukt eines Gens, das für NRP/B codiert; und/oder
    (ii) einem Translationsprodukt eines Gens, das für NRP/B codiert; und/oder
    (iii) einem Fragment oder mutanten, RNA-editierten oder chemisch modifizierten Derivat des Transcriptions- oder Translationsprodukts;

in einer Gehirngewebe- oder Liquorprobe von dem Patienten oder der Hochregulierung von NRP/B im frontalen Cortex relativ zum temporalen Cortex bei dem Patienten und Vergleichen der Konzentration mit einem Referenzwert, der einen bekannten Krankheits- oder Gesundheitszustand darstellt, wodurch die Alzheimer-Krankheit bei dem Patienten diagnostiziert oder prognostiziert wird oder bestimmt wird, ob der Patient ein erhöhtes Risiko hat, Alzheimer-Krankheit zu entwickeln.

2. Verfahren zur Überwachung des Fortschritts der Alzheimer-Krankheit bei einem Patienten, umfassend das Bestimmen der Hochregulierung von:

    (i) einem Transcriptionsprodukt eines Gens, das für NRP/B codiert; und/oder
    (ii) einem Translationsprodukt eines Gens, das für NRP/B codiert; und/oder
    (iii) einem Fragment oder mutanten, RNA-editierten oder chemisch modifizierten Derivat des Transcriptions- oder Translationsprodukts;

in einer Gehirngewebe- oder Liquorprobe von dem Patienten oder der Hochregulierung von NRP/B im frontalen Cortex relativ zum temporalen Cortex bei dem Patienten und Vergleichen der Konzentration mit einem Referenzwert, der einen bekannten Krankheits- oder Gesundheitszustand darstellt, wodurch der Fortschritt der Alzheimer-Krankheit bei dem Patienten überwacht wird.

3. Verfahren zur Bewertung einer Behandlung auf Alzheimer-Krankheit, umfassend das Bestimmen der Hochregulierung von:

    (i) einem Transcriptionsprodukt eines Gens, das für NRP/B codiert; und/oder
    (ii) einem Translationsprodukt eines Gens, das für NRP/B codiert; und/oder
    (iii) einem Fragment oder mutanten, RNA-editierten oder chemisch modifizierten Derivat des Transcriptions- oder Translationsprodukts;

in einer Gehirngewebe- oder Liquorprobe von dem Patienten oder der Hochregulierung von NRP/B im frontalen Cortex relativ zum temporalen Cortex bei dem Patienten, der auf Alzheimer-Krankheit behandelt wird, und Vergleichen der Konzentration mit einem Referenzwert, der einen bekannten Krankheits- oder Gesundheitszustand darstellt, wodurch die Behandlung auf Alzheimer-Krankheit bewertet wird.

**4.** Verfahren gemäß einem der Ansprüche 1 bis 3, wobei der Referenzwert der Referenzwert einer Konzentration von

(i) einem Transcriptionsprodukt eines Gens, das für NRP/B codiert; und/oder
(ii) einem Translationsprodukt eines Gens, das für NRP/B codiert; und/oder
(iii) einem Fragment oder mutanten, RNA-editierten oder chemisch modifizierten Derivat des Transcriptions- oder Translationsprodukts;

in einer Gehirngewebe- oder Liquorprobe von einem Patienten, der nicht an Alzheimer-Krankheit leidet, ist.

**5.** Verfahren gemäß einem der Ansprüche 1 bis 4, wobei eine Erhöhung in einem Transcriptionsprodukt des Gens, das für NRP/B codiert, und/oder in einem Translationsprodukt eines Gens, das für NRP/B codiert, in einer Gehirngewebe- oder Liquorprobe von dem Patienten relativ zu einem Referenzwert, der einen bekannten Gesundheitszustand darstellt, eine Diagnose oder Prognose oder ein erhöhtes Risiko von Alzheimer-Krankheit bei dem Patienten anzeigt.

**6.** Verfahren gemäß einem der Ansprüche 1 bis 5, das weiterhin das Vergleichen der Konzentration von

(i) einem Transcriptionsprodukt eines Gens, das für NRP/B codiert; und/oder
(ii) einem Translationsprodukt eines Gens, das für NRP/B codiert; und/oder
(iii) einem Fragment oder mutanten, RNA-editierten oder chemisch modifizierten Derivat des Transcriptions- oder Translationsprodukts; in einer Reihe von Gehirngewebe- oder Liquorproben, die im Laufe der Zeit von dem Patienten genommen wurden, umfasst.

**7.** Assay zum Suchen nach einem Modulator der Alzheimer-Krankheit durch Screening von einer oder mehreren Substanzen, die aus der Gruppe ausgewählt sind, die aus Folgenden besteht:

(i) einem Gen, das für NRP/B codiert; und/oder
(ii) einem Transcriptionsprodukt eines Gens, das für NRP/B codiert; und/oder
(iii) einem Translationsprodukt eines Gens, das für NRP/B codiert; und/oder
(iv) einem Fragment oder mutanten, RNA-editierten oder chemisch modifizierten Derivat von (i) bis (iii);

wobei das Verfahren Folgendes umfasst:

a) In-Kontakt-Bringen einer Zelle mit einer Testverbindung;
b) Messen der Konzentration von einer oder mehreren Substanzen, die in (i) bis (iv) genannt sind;
c) Messen der Konzentration von einer oder mehreren Substanzen, die in (i) bis (iv) genannt sind, in einer Kontrollzelle, die nicht mit der Testverbindung in Kontakt gebracht wurde; und
d) Vergleichen der Konzentrationen der Substanz in den Zellen der Schritte (b) und (c), wobei eine Änderung der Konzentration der Substanzen in den in Kontakt gebrachten Zellen anzeigt, dass die Testverbindung ein Modulator der Alzheimer-Krankheit ist.

**8.** Verfahren zum Suchen nach einem Modulator der Alzheimer-Krankheit durch Screening von einer oder mehreren Substanzen, die aus der Gruppe ausgewählt sind, die aus Folgenden besteht:

(i) einem Gen, das für NRP/B codiert; und/oder
(ii) einem Transcriptionsprodukt eines Gens, das für NRP/B codiert; und/oder
(iii) einem Translationsprodukt eines Gens, das für NRP/B codiert; und/oder
(iv) einem Fragment oder mutanten, RNA-editierten oder chemisch modifizierten Derivat von (i) bis (iii);

wobei das Verfahren Folgendes umfasst:

a) Verabreichen einer Testverbindung an ein Versuchstier mit Ausnahme des Menschen, das dafür prädisponiert ist, die Alzheimer-Krankheit zu entwickeln, oder bereits Alzheimer-Krankheit entwickelt hat in Bezug auf die Substanzen, die in (i) bis (iv) genannt sind;
b) Messen der Konzentration von einer oder mehreren Substanzen, die in (i) bis (iv) genannt sind;
c) Messen der Konzentration von einer oder mehreren Substanzen, die in (i) bis (iv) genannt sind, bei einem passenden Kontrolltier mit Ausnahme des Menschen, das dafür prädisponiert ist, die Alzheimer-Krankheit zu entwickeln, oder bereits Alzheimer-Krankheit entwickelt hat in Bezug auf die Substanzen, die in (i) bis (iv) genannt sind, wobei dem Tier keine solche Testverbindung verabreicht wurde;

d) Vergleichen der Konzentrationen der Substanz in den Tieren der Schritte (b) und (c), wobei eine Änderung der Konzentration der Substanzen in dem Versuchstier mit Ausnahme des Menschen anzeigt, dass die Testverbindung ein Modulator der Alzheimer-Krankheit ist.

**9.** Verfahren gemäß Anspruch 8, wobei das Versuchstier mit Ausnahme des Menschen und/oder das Kontrolltier mit Ausnahme des Menschen ein rekombinantes Tier ist, das NRP/B oder ein Fragment davon oder ein mutantes, RNA-editiertes oder chemisch modifiziertes Derivat davon unter der Kontrolle eines Transcriptionskontrollelements exprimiert, das nicht das native Transcriptionskontrollelement des NRP/B-Gens ist.

**10.** Verwendung eines Antikörpers, der gegenüber einem Immunogen spezifisch immunreaktiv ist, wobei das Immunogen ein Translationsprodukt eines Gens, das für NRP/B codiert, oder ein Fragment davon ist, zum Nachweis des pathologischen Zustands einer Zelle in einer Gehirngewebe- oder Liquorprobe von einem Patienten, der das immunocytochemische Anfärben der Zelle mit dem Antikörper umfasst, wobei ein veränderter Grad der Anfärbung im Vergleich zu einer Zelle, die einen bekannten Gesundheitszustand repräsentiert, einen pathologischen Zustand der Zelle anzeigt und wobei sich der pathologische Zustand auf die Alzheimer-Krankheit bezieht.

## Revendications

**1.** Procédé de diagnostic ou de pronostic de la maladie d'Alzheimer chez un sujet, ou de détermination si un sujet est hautement susceptible de développer la maladie d'Alzheimer, comprenant l'étape consistant à déterminer la régulation positive :

(i) d'un produit de transcription d'un gène codant pour NRP/B ; et/ou
(ii) d'un produit de traduction d'un gène codant pour NRP/B ; et/ou
(iii) d'un dérivé à ARN chimiquement modifié ou édité de fragment ou de mutant dudit produit de transcription ou de traduction ;

dans un échantillon de tissus cérébraux ou de liquide céphalorachidien provenant dudit sujet ou la régulation positive de NRP/B dans le cortex frontal par rapport au cortex temporal provenant dudit sujet et en comparant ladite concentration à une valeur de référence représentant un état pathologique ou non pathologique connu, diagnostiquant ou pronostiquant ainsi la maladie d'Alzheimer chez ledit sujet, ou en déterminant si ledit sujet est hautement susceptible de développer la maladie d'Alzheimer.

**2.** Procédé de surveillance de la progression de la maladie d'Alzheimer chez un sujet, comprenant l'étape consistant à déterminer la régulation positive :

(i) d'un produit de transcription d'un gène codant pour NRP/B ; et/ou
(ii) d'un produit de traduction d'un gène codant pour NRP/B ; et/ou
(iii) d'un dérivé à ARN chimiquement modifié ou édité de fragment ou de mutant dudit produit de transcription ou de traduction,

dans un échantillon de tissus cérébraux ou de liquide céphalorachidien provenant dudit sujet ou la régulation positive de NRP/B dans le cortex frontal par rapport au cortex temporal provenant dudit sujet et en comparant ladite concentration à une valeur de référence représentant un état pathologique ou non pathologique connu, surveillant ainsi la progression de la maladie d'Alzheimer chez ledit sujet.

**3.** Procédé d'évaluation d'un traitement pour la maladie d'Alzheimer, comprenant l'étape consistant à déterminer la régulation positive :

(i) d'un produit de transcription d'un gène codant pour NRP/B ; et/ou
(ii) d'un produit de traduction d'un gène codant pour NRP/B ; et/ou
(iii) d'un dérivé à ARN chimiquement modifié ou édité de fragment ou de mutant dudit produit de transcription ou de traduction ;

dans un échantillon de tissus cérébraux ou de liquide céphalorachidien provenant d'un sujet ou la régulation positive de NRP/B dans le cortex frontal par rapport au cortex temporal provenant dudit sujet traité pour la maladie d'Alzheimer et en comparant ladite concentration à une valeur de référence représentant un état pathologique ou non pathologique connu, évaluant ainsi ledit traitement pour la maladie d'Alzheimer.

4. Procédé selon l'une quelconque des revendications 1 à 3, dans lequel ladite valeur de référence est celle correspondant à une concentration :

(i) d'un produit de transcription d'un gène codant pour NRP/B ; et/ou
(ii) d'un produit de traduction d'un gène codant pour NRP/B ; et/ou
(iii) d'un dérivé à ARN chimiquement modifié ou édité de fragment ou de mutant dudit produit de transcription ou de traduction ;

dans un échantillon de tissus cérébraux ou de liquide céphalorachidien provenant d'un sujet ne souffrant pas de la maladie d'Alzheimer.

5. Procédé selon l'une quelconque des revendications 1 à 4, dans lequel une augmentation dans un produit de transcription du gène codant pour NRP/B et/ou dans un produit de traduction d'un gène codant pour NRP/B dans un échantillon de tissus cérébraux ou de liquide céphalorachidien, provenant dudit sujet par rapport à une valeur de référence représentant un état non pathologique connu, indique un diagnostic, un pronostic ou un risque accru de la maladie d'Alzheimer chez ledit sujet.

6. Procédé selon l'une quelconque des revendications 1 à 5, comprenant en outre l'étape consistant à comparer une concentration :

(i) d'un produit de transcription d'un gène codant pour NRP/B ; et/ou
(ii) d'un produit de traduction d'un gène codant pour NRP/B ; et/ou
(iii) d'un dérivé à ARN chimiquement modifié ou édité de fragment ou de mutant dudit produit de transcription ou de traduction ;

dans une série d'échantillons de tissus cérébraux ou de liquide céphalorachidien prélevés sur ledit sujet pendant une durée donnée.

7. Dosage pour cribler un modulateur de la maladie d'Alzheimer d'une ou plusieurs substances choisies dans le groupe constitué par :

(i) un gène codant pour NRP/B ; et/ou
(ii) un produit de transcription d'un gène codant pour NRP/B ; et/ou
(iii) un produit de traduction d'un gène codant pour NRP/B ; et/ou
(iv) un dérivé à ARN chimiquement modifié ou édité de fragment ou de mutant de (i) à (iii), ledit procédé comprenant les étapes consistant à :

(a) mettre en contact une cellule avec un composé d'essai ;
(b) mesurer les concentrations d'une ou plusieurs des substances mentionnées en (i) à (iv) ;
(c) mesurer les concentrations d'une ou plusieurs des substances mentionnées en (i) à (iv) dans une cellule témoin n'ayant pas été mise en contact avec ledit composé d'essai ; et
(d) comparer les concentrations de la substance dans les cellules des étapes (b) et (c), où une modification des concentrations des substances dans les cellules en contact indique que le composé d'essai est un modulateur de la maladie d'Alzheimer.

8. Procédé pour cribler un modulateur de la maladie d'Alzheimer d'une ou plusieurs substances choisies dans le groupe constitué par :

(i) un gène codant pour NRP/B ; et/ou
(ii) un produit de transcription d'un gène codant pour NRP/B ; et/ou
(iii) un produit de traduction d'un gène codant pour NRP/B ; et/ou
(iv) un dérivé à ARN chimiquement modifié ou édité de fragment ou de mutant de (i) à (iii), ledit procédé comprenant les étapes consistant à :

(a) administrer un composé d'essai à un animal de laboratoire hormis l'Homme qui est prédisposé à développer ou qui a déjà développé la maladie d'Alzheimer en ce qui concerne les substances mentionnées en (i) à (iv) ;

(b) mesurer les concentrations d'une ou plusieurs des substances mentionnées en (i) à (iv) ;

(c) mesurer les concentrations d'une ou plusieurs des substances mentionnées en (i) ou (iv) chez un animal témoin correspondant hormis l'Homme qui est prédisposé à développer ou qui a déjà développé la maladie d'Alzheimer en ce qui concerne les substances mentionnées en (i) à (iv), aucun composé d'essai n'ayant été administré à cet animal ;

(d) comparer les concentrations de la substance chez les animaux des étapes (b) et (c), où une modification de la concentration des substances chez l'animal de laboratoire hormis l'Homme indique que le composé d'essai est un modulateur de la maladie d'Alzheimer.

9.  Procédé selon la revendication 8, dans lequel ledit animal de laboratoire hormis l'Homme et/ou ledit animal témoin hormis l'Homme est un animal recombiné qui exprime H NRP/B, ou un de ses dérivés à ARN chimiquement modifié ou édité d'un de ses fragments ou mutants régulé par un composant témoin transcriptionnel qui n'est pas le composant témoin transcriptionnel du gène NRP/B naturel.

10.  Utilisation d'un anticorps spécifiquement immunoréactif avec un agent immunogène, dans laquelle ledit agent immunogène est un produit de traduction d'un gène codant pour NRP/B, ou un de ses fragments, pour détecter l'état pathologique d'une cellule dans un échantillon de tissus cérébraux ou de liquide céphalorachidien provenant d'un sujet, comprenant les étapes consistant à colorer immunocytochimiquement ladite cellule avec ledit anticorps, où un degré de coloration modifié par rapport à une cellule représentant un état non pathologique connu, indique un état pathologique de ladite cellule, et où ledit état pathologique concerne la maladie d'Alzheimer.

## Fig. 1: Identification of genes involved in Alzheimer's Disease pathology

EP 1 379 882 B1

Fig. 2: Verification of Differential Expression of NRP/B by Quantitative RT-PCR

a)

Fluorescence

F

T

Cycles

b)

Fluorescence

F

T

Cycles

# Table 1:

| SAMPLE | Δ (fold) |
| --- | --- |
| patient 1 (#12) | 3.35 |
| patient 2 (#16) | 4.42 |
| patient 3 (#10) | 13.07 |
| patient 4 (#11) | 4.67 |
| patient 5 (#14) | 11.26 |
| control 1 (#10) | 1.23 |
| control 2 (#11) | 0.58 |
| control 3 (# 5) | 1.10 |
| control 4 (# 4) | 2.17 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6150173 A

**Non-patent literature cited in the description**

- **VICKERS et al.** *Progress in Neurobiology,* 2000, vol. 60, 139-165
- **TERRY et al.** *Annals of Neurology,* 1981, vol. 10, 184-192
- **KIM et al.** *Journal of Cell Biology,* 1998, vol. 141, 553-566
- **SAMBROOK ; RUSSELL.** Molecular Cloning: A Laboratory Manual. Cold Spring Harbor Laboratory Press, 2000
- **HARLOW ; LANE.** Antibodies: A Laboratory Manual. Cold Spring Harbor Laboratory Press, 1999
- **HARLOW et al.** Antibodies, A Laboratory Manual. Cold Spring Harbor Laboratory Press
- Brain Biochemistry and Brain Disorders. Oxford University Press, 1992, 4
- **DIATCHENKO et al.** *Proc Natl Acad Sci USA,* 1996, vol. 93, 6025-30
- **WANG ; BROWN.** *Proc Natl Acad Sci USA,* 1991, vol. 88, 11505-9